(19) Europäisches Patentamt
European Patent Office
Office européen des brevets

(11) **EP 1 151 304 B1**

(12) **EUROPÄISCHE PATENTSCHRIFT**

(45) Veröffentlichungstag und Bekanntmachung des Hinweises auf die Patenterteilung:
**19.09.2007  Patentblatt 2007/38**

(21) Anmeldenummer: **00903642.7**

(22) Anmeldetag: **27.01.2000**

(51) Int Cl.:
***G01N 33/68*** *(2006.01)*

(86) Internationale Anmeldenummer:
**PCT/EP2000/000602**

(87) Internationale Veröffentlichungsnummer:
**WO 2000/045176 (03.08.2000 Gazette 2000/31)**

(54) **VERFAHREN ZUM NACHWEIS VON N-TERMINALEM proBNP**

METHOD OF IDENTIFYING N-TERMINAL proBNP

PROCEDE D'IDENTIFICATION DE proBNP N-TERMINAL

(84) Benannte Vertragsstaaten:
**AT BE CH CY DE DK ES FI FR GB GR IE IT LI LU MC NL PT SE**

(30) Priorität: **29.01.1999  DE 19903489
12.03.1999  DE 19911044**

(43) Veröffentlichungstag der Anmeldung:
**07.11.2001  Patentblatt 2001/45**

(60) Teilanmeldung:
**06010440.3 / 1 698 901**

(73) Patentinhaber: **Roche Diagnostics GmbH
68305 Mannheim (DE)**

(72) Erfinder:
• **KARL, Johann
D-82380 Peissenberg (DE)**
• **LILL, Helmut
D-82407 Wielenbach (DE)**
• **STAHL, Peter
D-82347 Bernried (DE)**
• **KRUEGER, Kerstin
D-81377 München (DE)**
• **BORGYA, Anneliese
D-82327 Tutzing (DE)**
• **GALLUSSER, Andreas
D-82377 Penzberg (DE)**

(56) Entgegenhaltungen:
**WO-A-89/12069          WO-A-93/24531**

• **HUNT P J ET AL: "IMMUNOREACTIVE AMINO-TERMINAL PRO-BRAIN NATRIURETIC PEPTIDE (NT-PROBNP): A NEW MARKER OF CARDIAC IMPAIRMENT" CLINICAL ENDOCRINOLOGY,GB, BLACKWELL SCIENTIFIC PUBLICATIONS, OXFORD, Bd. 47, 1997, Seiten 287-296, XP000913471 ISSN: 0300-0664**
• **DAGGUBATI, S. ET AL.: "Adrenomedullin, endothelin, neuropeptide Y, atrial, brain, and C-natriuretic prohormone peptides compared as early heart failure indicators" CARDIOVASCULAR RESEARCH, Bd. 36, 1997, Seiten 246-255, XP000913576**
• **HUNT P J ET AL: "THE AMINO-TERMINAL PORTION OF PRO-BRAIN NATRIURETIC PEPTIDE (PRO-BNP) CIRCULATES IN HUMAN PLASMA" BIOCHEMICAL AND BIOPHYSICAL RESEARCH COMMUNICATIONS,US,ACADEMIC PRESS INC. ORLANDO, FL, Bd. 214, Nr. 3, 1995, Seiten 1175-1183, XP000907386 ISSN: 0006-291X in der Anmeldung erwähnt**
• **HUNT P J ET AL: "THE ROLE OF THE CIRCULARION IN PROCESSING PRO-BRAIN NATRIURETIC PEPTIDE (PROBNP) TO AMINO-TERMINAL BNP AND BNP-32" PEPTIDES,US, ELMSFORD, Bd. 18, Nr. 10, 1997, Seiten 1475-1481, XP000913698 ISSN: 0196-9781**
• **HUGHES D. ET AL: "An immunoluminescent assay for N-terminal pro-brain natriuretic peptide: development of a test for left ventricular dysfunction." CLINICAL SCIENCE, Bd. 96, 1999, Seiten 373-380, Great Britain**

**Beschreibung**

Die vorliegende Erfindung betrifft ein Verfahren zum Nachweis von N-terminalem proBNP in einer Probe mit mindestens zwei Antikörpern, die verschiedene Epitope des N-terminalen proBNP erkennen. Das Verfahren kann zur Differenzierung bzw. Klassifizierung von Proben von Gesunden und Proben von Patienten der NYHA-Klassen I bis IV angewendet werden. Außerdem betrifft die Erfindung die Verwendung von rekombinantem N-terminalen proBNP als Standard in einem Verfahren zum Nachweis von N-terminalem proBNP, sowie Antikörper, die rekombinantes N-terminales proBNP erkennen, und deren Herstellung.

Herzinsuffizienz ist ein weit verbreitetes Phänomen insbesondere in der westlichen Welt. Unter Herzinsuffizienz versteht man gemäß Roche Lexikon Medizin (1993, Urban & Schwarzenberg) das akute oder chronische Unvermögen des Herzens, bei Belastung oder schon im Ruhezustand den für den Stoffwechsel erforderlichen Blutauswurf aufzubringen bzw. den venösen Rückfluss aufzunehmen (sogenannter Back-ward- und Forward-Failure). Es liegt also eine Schwäche der Pumpenfunktion vor. Die Ursachen für Herzinsuffizienz sind vielschichtig. Unter anderem sind hier entzündliche und degenerative Veränderungen des Herzmuskels, koronare Durchblutungsstörung, Herzinfarkt und Verletzungen zu nennen. Dies führt zu Veränderungen am peripheren Blutkreislauf, Störung der Atmung, der Nierenfunktion und des Elektrolytstoffwechsels (Ödeme) und zu verminderter Leistungsfähigkeit der Skelettmuskulatur.

Gemäß der New York Heart Association (NYHA) wird Herzinsuffizienz anhand von körperlichen Belastungstests in sogenannte NYHA-Klassen eingeteilt: I bedeutet völlige Beschwerdefreiheit bei normaler körperlicher Belastung, II bedeutet leichte Einschränkung der körperlichen Belastbarkeit, III bedeutet starke Einschränkung der Belastbarkeit, IV bedeutet, daß bei jeder körperlichen Tätigkeit eine Zunahme der meist auch in Ruhe bestehenden Insuffizienzzeichen stattfindet.

Zu einer effektiven medikamentösen Behandlung von Herzinsuffizienz mittels Glykosiden, Vasodilatoren, ACE-Hemmern und/oder β-Blockern ist es erforderlich, zuvor das Vorliegen einer Herzinsuffizienz genau zu diagnostizieren und möglichst auch nach ihrem Schweregrad zu klassifizieren und zusätzlich den Verlauf der Behandlung zu monitoren.

Im Stand der Technik werden einige Serummarker zur frühen Diagnose von Herzinsuffizienz wie beispielsweise ANP (N-terminal atrial natriuretic peptide hormon) und proANP, CNP (C-natriuretic peptide), Adrenomedullin, Neuropeptid Y, Endothelin und BNP (brain natriuretic peptide) diskutiert. ANP und proANP sind prinzipiell als Marker zur Diagnose von Herzinsuffizienz geeignet, besitzen jedoch eine geringe Stabilität bzw. kurze Halbwertzeit im Blut, was diagnostische Messungen erschwert (Clin. Sci. 95(3) (1998), 235-239; Cleland et al., Heart 75 (1996), 410-413).

Ein häufig zitierter und als aussagekräftig angesehener Marker ist das BNP (brain natriuretic peptide). BNP wurde ursprünglich im Gehirn von Schweinen identifiziert. Es handelt sich dabei um ein Herzhormon, das strukturelle und funktionelle Ähnlichkeit mit ANP (atrial natriuretic peptide) besitzt (Sudoh et al., Nature 332 (1988), 78-81). Das humane BNP, das aus 32 Aminosäuren besteht, wird vor allem von den Herzventrikeln sekretiert und zirkuliert im humanen Blutplasma. Der Einsatz von BNP als diagnostischer Marker ist beispielsweise aus der EP-A-0 542 255 bekannt. BNP besitzt eine intramolekulare Disulfidbrücke und ist als Analyt vermutlich aufgrund seiner physiologischen Funktion als Hormon, das schnell wieder abgebaut werden muß, nicht sehr stabil und daher als diagnostischer Marker nur beschränkt geeignet (Masuta et al., Clin. Chem. Vol. 44 No. 6 Supplement A (1998), 130; Tsuji et al., Clin. Chem. 40 (1994), 672).

Das Vorläufer-Molekül von BNP, das proBNP, besteht aus 108 Aminosäuren, von denen die zuvor beschriebenen 32 C-terminalen Aminosäuren (77-108), die als BNP bezeichnet werden, die eigentliche hormonelle Wirkung entfalten. Die vom Precursor abgespaltenen N-terminalen Aminosäuren 1-76 werden als N-terminales proBNP bezeichnet. Neben BNP (77-108) zirkuliert auch N-terminales proBNP und weitere Abbauprodukte 1-76) im Plasma (Hunt et al., Biochem. Biophys. Res. Com. 214 (1995), 1175-1183), so daß N-terminales proBNP ebenfalls als Marker für Herzinsuffizienz in Frage kommt. Ob auch das Vorläufer-Molekül proBNP im Plasma vorkommt, ist nicht eindeutig geklärt. Es wird aber beschrieben (Hunt et al., Peptides. Vol. 18, No. 10 (1997), 1475-1481), daß eine geringe Ausschüttung von proBNP (1-108) in Plasma nachweisbar ist, aber aufgrund eines sehr schnellen partiellen Abbaus am N-terminalen Ende einige Aminosäuren fehlen. Dieses Molekül wird in der Literatur als High Molecluar Weight-BNP bezeichnet.

In der WO 93/24531 (US 5,786,163) wird ein immunologisches Verfahren zum Nachweis von N-terminalem proBNP und dazu verwendete Antikörper beschrieben. Zur Gewinnung der Antikörper werden hier einzelne synthetisch hergestellte Peptide aus der Sequenz von N-terminalem proBNP verwendet. Prinzipiell ist zwar die Gewinnung von Antikörpern mittels Peptidimmunisierung möglich, doch ist die Affinität zum Gesamtmolekül im allgemeinen zu niedrig, um die erforderliche Sensitivität in einem Testverfahrens zu erreichen. Darüberhinaus besteht die Gefahr, daß bei Verwendung von Peptiden Antikörper gewonnen werden, die beispielsweise den C-Terminus des Peptids erkennen und somit nur dieses Bruchstück des Gesamtmoleküls binden können. Daraus folgt, daß diese Antikörper das Gesamtmolekül nicht oder nur schwach binden können. In der WO 93/24531 werden polyklonale Antikörper gegen ein einziges vom N-terminalen proBNP abgeleitetes Peptid hergestellt. Es wird gezeigt, daß die erzeugten Antikörper zwar das Immunisierungspeptid (Aminosäuren 47-64) im kompetitiven Testformat binden. Es wird jedoch nicht gezeigt, daß die Antikörper in der Lage sind, natives N-terminales proBNP als Gesamtmolekül in einer Probe zu binden. Der in der WO 93/24531 beschriebene Sandwichtest in einer Probe kann darüberhinaus nicht wie beschrieben durchgeführt werden, da kein geeignetes Standardmaterial zur Verfügung stand und keine Antikörper gegen zwei verschiedene Epitope vorgelegen haben.

Ein weiteres Problem im Stand der Technik ist die Sensitivität des Tests. Anhand des in der WO 93/24531 durchgeführten kompetitiven Tests, bei dem das Peptid 47-64 in markierter Form als Tracer mit einer Probe bzw. mit dem unmarkierten Peptid-Standard 47-64 um die Bindung an polyklonale Antikörper aus Kaninchenserum kompetieren, wird nach 48-stündiger Inkubation nur eine sehr mäßige Kompetition erreicht, aus der bestenfalls eine untere Nachweisgrenze von etwa 250 fmol/mln abgeleitet werden kann. Dies ist weder für die Differenzierung von Gesunden und Patienten mit einer Herzinsuffizienz, noch für eine differenzierte Klassifizierung von Patientenproben nach Schweregrad der Herzinsuffizienz ausreichend. Außerdem sind die langen Inkubationszeiten des kompetitiven Tests für routinemäßige Messungen der Proben im automatisierten Labor nicht akzeptabel.

Von Hunt et al. (Clinical Endocrinology 47 (1997), 287-296) wird ebenfalls ein kompetitiver Test zum Nachweis von N-terminalem proBNP beschrieben. Hierzu muß die Plasmaprobe vor Vermessung aufwendig extrahiert werden, was die Gefahr birgt, daß der Analyt dabei zerstört wird und Fehlmessungen auftreten. Das hier verwendete Antiserum wird analog zur WO 93/24531 durch Immunisierung mit einem synthetischen Peptid erzeugt. Bei Hunt et al. wird das Antiserum durch Immunisierung mit den N-terminalen proBNP-Aminosäuren 1-13 erzeugt, als Standard wird das Peptid von Aminosäure 1-21 eingesetzt. Auch in diesem Test muß eine lange Inkubationszeit in Kauf genommen werden. Nach 24-stündiger Inkubation wird eine untere Nachweisgrenze von 1,3 fmol/ml erreicht.

Im Stand der Technik existiert somit kein Verfahren zum Nachweis von N-terminalem proBNP, das bei kurzen Inkubationsdauern einen zuverlässigen, sensitiven Nachweis von nativem N-terminalem proBNP ermöglicht.

Aufgabe war es daher, ein Verfahren zum Nachweis von N-terminalem proBNP in einer Probe bereitzustellen, das die aufgeführten Nachteile des Standes der Technik weitestgehend vermeidet. Insbesondere sollte eine hohe Sensitivität im Test erreicht Aufgabe war es daher, ein Verfahren zum Nachweis von N-terminalem proBNP in einer Probe bereitzustellen, das die aufgeführten Nachteile des Standes der Technik weitestgehend vermeidet. Insbesondere sollte eine hohe Sensitivität im Test erreicht werden können, damit eine Differenzierung der Patientenproben von Gesunden und Proben von Patienten der NYHA-Klassen I bis IV erfolgen kann.

Die Aufgabe wird gelöst durch das in den Ansprüchen näher definierte Verfahren zum Nachweis von N-terminalem proBNP in einer Probe. Das Verfahren ist dadurch gekennzeichnet, daß mindestens zwei Antikörper, die verschiedene Epitope des N-terminalen proBNP erkennen, verwendet werden, wobei Antikörper durch Immunisieren eines geeigneten Organismus mit rekombinantem N-terminalen proBNP erhalten werden und die Antikörper im Bereich von Aminosäure 10-50 des N-terminalen proBNP binder. Das wesentliche am erfindungsgemäßen Verfahren ist, daß natives N-terminales proBNP in einer Probe erfasst wird. Das heißt, die Antikörper müssen in der Lage sein, das intakte Molekül und eventuell vorkommendes ungespaltenes proBNP (1-108) und möglichst auch proteolytisch angedaute Bruchstücke in einer Probe zu erkennen und spezifisch zu binden. Im Verfahren werden mindestens zwei verschiedene Antikörper eingesetzt, die verschiedene Epitope des N-terminalen proBNP binden. Die Epitope können linear oder sogenannte Konformationsepitope sein. Bevorzugt sind die Epitope so lokalisiert; daß beide Antikörper gleichzeitig binden können und nicht zu weit voneinander entfernt liegen.

Da die erfindungsgemäße Methode es nicht erlaubt zwischen N-terminalem proBNP, proBNP und nahe verwandten Peptiden (Abbauprodukten) zu differenzieren, wird im folgenden unter NT-proBNP die Gesamtheit aller im Testverfahren erkannter Peptide, insbesondere das bekannte N-terminale proBNP (1-76), verstanden.

Unter dem Begriff "Epitop" wird erfindungsgemäß die Bindungsstelle auf einem immunologischen Bindepartner wie beispielsweise einem Antigen verstanden, an den ein Antikörper spezifisch bindet. Ein "Epitop" wird meist eindeutig durch 6 bis 8 Aminosäuren definiert. Der Bindepartner entspricht erfindungsgemäß dem N-terminalen proBNP beziehungsweise einer Teilsequenz davon. Das Epitop, an den der Antikörper Mittels der beiden Antikörper unterschiedlicher Spezifität ist es möglich, statt der kompetitiven langwierigen Testführung des Standes der Technik ein schnelleres Verfahren zum Nachweis des Analyten durchzuführen Das erfindungsgemäße Nachweisverfahren kann mittels homogener oder heterogener Testführung erfolgen. Bevorzugt ist die heterogene Testführung und besonders bevorzugt das dem Fachmann geläufige Sandwich-Verfahren.

Bevorzugt wird ein solches Verfahren zur Bestimmung des N-terminalen proBNP in folgenden Schritten durchgeführt:

a) Umsetzen der Probe mit einem für N-terminales proBNP spezifischen ersten Antikörper, der eine mit einer Festphase bindefähige Gruppe trägt, über die die Bindung an eine Festphase erfolgen kann, oder Umsetzen der Probe mit einem für N-terminales proBNP spezifischen ersten Antikörper, der bereits an eine Festphase gebunden ist,

b) Umsetzen dieser Lösung mit dem zweiten Antikörper, der ein anderes Epitop von NT-proBNP erkennt als der erste Antikörper, und der eine Markierung trägt

c) Bindung des gebildeten Immunkomplexes an eine Festphase, wobei die Festphase bereits in Schritt a) vorhanden sein kann

d) Trennung der festen von der flüssigen Phase
e) Detektion der Markierung in einer oder beiden Phasen.

Bei quantitativer Bestimmung wird die gleiche Messung mit einer definierten Menge an N-terminalem proBNP als Standard durchgeführt und nach Bestimmung der Probe als Schritt f) ein Vergleich der Messwerte des Standards mit dem Wert der Probe und die Quantifizierung durchgeführt.

Unter dem Begriff "Antikörper" werden im Sinne der Erfindung mono- oder polyklonale, chimäre oder humanisierte oder andere durch gentechnologische Modifikationen erhältlichen Antikörper sowie sämtliche dem Fachmann bekannten Fragmente wie F(ab')$_2$, Fab' oder Fab-Fragmente verstanden. Lediglich die immunologische spezifische Bindefähigkeit an N-terminales proBNP muß gewährleistet sein.

Der erste für N-terminales proBNP spezifische Antikörper kann entweder direkt an die Festphase gebunden sein, oder die Bindung an die Festphase erfolgt indirekt über ein spezifisches Bindungssystem. Die direkte Bindung dieses Antikörpers an die Festphase erfolgt nach dem Fachmann bekannten Methoden, beispielsweise adsorptiv. Wird die Bindung indirekt über ein spezifisches Bindungssystem durchgeführt, so ist der erste Antikörper ein Konjugat, das aus einem Antikörper gegen N-terminales proBNP und einem Reaktionspartner eines spezifischen Bindungssystems besteht. Unter einem spezifischen Bindungssystem werden hier zwei Partner verstanden die spezifisch miteinander reagieren können. Das Bindungsvermögen kann dabei auf einer immunologischen Reaktion oder auf einer anderen spezifischen Reaktion beruhen. Bevorzugt wird als spezifisches Bindungssystem eine Kombination von Biotin und Avidin oder Biotin und Streptavidin verwendet. Weitere bevorzugte Kombinationen sind Biotin und Antibiotin. Hapten und Anti-Hapten, Fc-Fragment eines Antikörpers und Antikörper gegen dieses Fc-Fragment oder Kohlenhydrat und Lectin. Einer der Reaktionspartner des spezifischen Bindungssystems ist dann Teil des Konjugates.

Der andere Reaktionspartner des spezifischen Bindungssystems für den ersten Bindepartner liegt als Beschichtung der festen Phase vor. Bevorzugt wird hier Streptavidin oder Avidin verwendet. Die Bindung des anderen Reaktionspartners des spezifischen Bindungssystems an ein unlösliches Trägermaterial kann nach den üblichen, dem Fachmann bekannten Methoden vorgenommen werden. Hierbei ist sowohl eine kovalente als auch eine adsorptive Bindung geeignet.

Als Festphase geeignet sind Reagenzeläschen oder Mikrotiterplatten aus Polystyrol oder ähnlichen Kunststoffen, die an der Innenoberfläche mit einem Reaktionspartner des spezifischen Bindungssystems beschichtet sind. Weiterhin geeignet und besonders bevorzugt sind teilchenförmige Substanzen, wie beispielsweise Latexpartikel, magnetische Partikel, Molekularsiebmaterialien, Glaskörperchen, Kunststoffschläuche und dergleichen. Auch poröse, schichtförmige Träger wie Papier oder Nitrocellulose können als Träger verwendet werden. Besonders bevorzugt werden magnetische Kügelchen, sogenannte Beads verwendet, die wiederum mit dem entsprechenden Bindepartner des oben beschriebenen spezifischen Bindesystems beschichtet sind. Diese Mikropartikel können dann nach Ablauf der Testreaktion für die Durchführung der Nachweisreaktion beispielsweise durch Filtration, Zentrifugation oder im Falle der magnetischen Partikel durch einen Magneten von der flüssigen Phase getrennt werden.

Der zweite spezifische Antikörper erkennt ein anderes Epitop des N-terminalen proBNP als der erste Antikörper. Der Abstand der beiden Epitope auf dem Molekül muß so groß sein, daß die gleichzeitige Bindung der beiden Antikörper an das N-terminale proBNP ohne Einschränkung möglich ist, da ansonsten kein Sandwich-Komplex gebildet werden kann.

Die Detektion der spezifischen Bindereaktionen zwischen den Antikörpern gegen N-terminales proBNP und N-terminalem proBNP kann auf verschiedene Weise erfolgen. Im allgemeinen ist der zweite Antikörper markiert. Übliche Markierungen sind Chromogene, Fluorophore, zur Chemi- oder Elektrochemilumineszenz fähige Substanzen, Radioisotope, Haptene, Enzymmarkierungen oder Substanzen, die wiederum ein spezifisches Bindungspaar bilden können wie beispielsweise Biotin/Streptavidin. Der Nachweis des Immunkomplexes erfolgt dann anhand des Signals, das von der Markierung ausgesandt wird. Beispielsweise kann der zweite Antikörper mit dem Hapten Digoxigenin markiert sein. Dieses Hapten wird wiederum von einem weiteren, für Digoxigenin spezifischen Antikörper gebunden. Der für Digoxigenin spezifische Antikörper ist selbst beispielsweise mit einem Enzym wie Peroxidase markiert. Der letztendliche Nachweis erfolgt dann anhand der bei der Umsetzung der Peroxidase mit einem entsprechenden Substrat erfolgenden Farb- bzw. Extinktionsänderung.

Als Proben für die Durchführung des Verfahrens zum Nachweis von N-terminalem proBNP können alle dem Fachmann geläufigen biologischen Flüssigkeiten verwendet werden. Bevorzugt werden als Probe Körperflüssigkeiten wie Vollblut, Blutserum, Blutplasma, Urin oder Speichel, besonders bevorzugt wird Blutserum und -plasma eingesetzt.

Neben den sogenannten Naßtesten, bei denen die Testreagenzien in flüssiger Phase vorliegen, können auch alle gängigen Trockentestformate, die zum Nachweis von Antigenen, Haptenen, Peptiden, Proteinen, Antikörpern etc. geeignet sind, verwendet werden. Bei diesen Trockentests oder Teststreifen, wie sie beispielsweise in der EP-A-0 186 799 beschrieben sind, sind im allgemeinen alle Testkomponenten bis auf die zu untersuchende Probe auf einem Träger aufgebracht. Die Nachweisreaktion erfolgt, wenn der Teststreifen mit der flüssigen Probe in Kontakt gebracht wird.

Das erfindungsgemäße Verfahren zeichnet sich dadurch aus, daß die untere Nachweisgrenze für N-terminales proBNP unter 1 fmol/ml liegt (entspricht 1 pmol/l). Die erfindungsgemäß hohe Sensitivität von < 1 fmol/ml wird ohne lange Inkubationszeiten erreicht Insgesamt liegt die Dauer des Verfahrens bei einem Mikrotiterplattentest unter 2 Stunden, bevorzugt mit sensitiveren Nachweismethoden wie der Electrochemilumineszenz bei 15 Minuten. Eine Obergrenze für das Nachweisverfahren bezüglich der nachzuweisenden Konzentration existiert praktisch nicht. Die technologische Obergrenze wird im allgemeinen durch die verwendete Meßmethode vorgegeben. Das Verfahren weist prinzipiell auch sehr hohe Konzentrationen an N-terminalem proBNP nach.

Ein weiterer Vorteil des erfindungsgemäßen Verfahrens ist die gute Differenzierung der Proben von Patienten mit und ohne Herzinsuffizienz anhand der erhaltenen Messwerte. Das Nachweisverfahren ist so sensitiv, daß sogar eine Differenzierung zwischen Patienten ohne koronarer Erkrankung und Patienten mit einer nur schwach ausgeprägten oder langsam einsetzenden Herzinsuffizienz der NYHA-Klassen I und II erfolgen kann. Eine solch frühe Erkennung einer einsetzenden Herzinsuffizienz kann die Entscheidung zu einer frühzeitigen medikamentösen Behandlung beeinflussen und somit die Überlebensrate des Patienten deutlich verlängern.

Offenbart wird weiterhin rekombinant hergestelltes N-terminales proBNP. Unter N-terminalem proBNP wird der vom 108 Aminosäuren großen Vorläufermolekül proBNP abgespaltene N-terminale Teil verstanden, der aus den Aminosäuren 1-76 besteht. Unter N-terminalem proBNP werden auch Teile davon verstanden, die aufgrund von Abbaureaktionen dieses Moleküls im Blut vorkommen können

Im Stand der Technik ist bisher kein rekombinantes N-terminales proBNP bekannt, da dessen Herstellung aufgrund der kurzen Aminosäuresequenz nicht leicht möglich ist. Die chemische Synthese eines über 30 Aminosäuren großen Peptids ist aufgrund der auftretenden Fehlsequenzen und der stark abnehmenden Ausbeute pro Synthesezyklus keine Alternative zur rekombinanten Herstellung in einem Wirtsorganismus.

Für ein diagnostisches Nachweisverfahren wird jedoch immer ein Standard- oder Kontrollmaterial benötigt, mit dessen Hilfe zum einen die Bestimmung des Analyten quantitativ erfolgen kann und zum anderen die Funktionsfähigkeit des Tests überprüft werden kann.. Soll eine Quantifizierung erfolgen, so muß mittels einer Standardreihe eine definierte quantitative Eichung vorgenommen werden. Eine solche Eichung ist wiederum auch nur dann sinnvoll, wenn das als Standard verwendete Material sich im immunologischen Test gleich oder sehr ähnlich verhält wie der Analyt. Wesentlich dabei ist, daß der Standard eine ausreichend große strukturelle und insbesondere immunologische Ähnlichkeit zum Analyten besitzt, damit die Bindung des Standards an die Nachweisantikörper so erfolgt, wie sie dann auch beim nativen Molekül in der Probe stattfindet.

Ein solches Standardmaterial für ein Verfahren zum Nachweis von N-terminalem proBNP steht im Stand der Technik nicht zur Verfügung. Lediglich kurze synthetische Peptide werden beschrieben. Erfindungsgemäß ist es nun erstmals möglich gewesen, mit Hilfe der Gensynthese eine für N-terminales proBNP codierende DNA-Sequenz herzustellen und anschließend eine rekombinante Expression des N-terminalen proBNP in E. coli zu erreichen. Die Vorgehensweise ist in Beispiel 1 erläutert.

Gegenstand der Erfindung ist daher die Verwendung von rekombinantem N-terminalen proBNP als Standard in einem Verfahren zum Nachweis von N-terminalem proBNP in einer Probe mittels mindestens zweier Antikörper, die verschiedene Epitope des N-terminalen proBNP erkennen, wobei die Antikörper durch Immunisieren eines geeigneten Organismus mit rekombinantem N-terminalen proBNP erhalten werden und die Antikörper im Bereich von Aminosäure 10-50 des N-terminalen proBNP binden.

Für Immunisierungszwecke wurden im Stand der Technik bislang ebenfalls nur synthetische, vom N-terminalen proBNP abgeleitete kurze Peptide eingesetzt. Der Nachteil bei Peptidimmunisierungen ist, daß meist nur sehr niedrig af6ne Antikörper erhalten werden bzw. die gewonnenen Antikörper nur mit linearen Epitopen reagieren und das nativ gefaltete Antigen in der Probe nicht gebunden werden kann (siehe Beispiel 3).

Deshalb ist es wichtig, für Immunisierungen zur Herstellung von Antikörpern ein Immunogen zu verwenden, das zum letztendlich nachzuweisenden Analyten eine genügend große Ähnlichkeit aufweist Nur so ist gewährleistet, daß von den Antikörpern der native Analyt in der Probe mit hoher Affinität gebunden wird.

Offenbart wird daher auch die Verwendung von rekombinantem N-terminalem proBNP als Immunogen zur Herstellung von Antikörpern gegen N-terminales proBNP.

Ein weiterer Gegenstand der Erfindung sind Antikörper gegen rekombinantes N-terminales proBNP. Die Antikörper werden hergestellt durch Immunisieren eines geeigneten Organismus mit rekombinantem N-terminalen proBNP und sind dadurch gekennzeichnet, dass sie im Bereich von Aminosäure 10-50 des N-terminalen proBNP binden. Die Definition des Begriffes Antikörper entspricht der Definition in den Abschnitten über die Testführung. Die erfindungsgemäßen Antikörper erkennen bevorzugt spezifisch Epitope im N-terminalen Teil des 76 Aminosäuren großen N-terminalen proBNP, dabei bevorzugt im Bereich von Aminosäure 10 bis 66, besonders bevorzugt im Bereich von Aminosäure 10 bis 50 oder 10 bis 38. Sinnvoll ist es, wenn die von den Antikörpern erkannten Epitope so lokalisiert sind, daß auch N-terminales proBNP, das in einer Probe bereits von den Enden her proteolytisch angedaut ist, diese Epitope noch enthält. Somit ist die Stabilität des Analyten in der Probe eher zweitrangig. Die Epitope in den bevorzugten Bereichen des N-terminalen proBNP können linear oder als Konformationsepitope vorliegen.

Ein bevorzugter Gegenstand der Erfindung sind daher monoklonale Antikörper die von den Zellinien MAK M 10.1.1 und MAK M 13.4.14, hinterlegt und eingegangen am 26.01.1999 bei der DSMZ - Deutsche Sammlung von Mikroorganismen und Zellkulturen GmbH, Braunschweig, Deutschland, produziert werden. Bei den Antikörpern, die von diesen beiden Zellinien produziert werden, handelt es sich um Antikörper vom IgG-Typ. Die Zellinien M 10.1.11 und M 13.4.14 sind ebenfalls Gegenstand der Erfindung.

Ebenfalls ein Gegenstand der Erfindung sind Antikörper, die in äquivalenter Weise wie die von den Zellinien M 10.1.11 und M 13.4.14 produzierten mit N-terminalem proBNP spezifisch bindefähig sind. Unter dem Begriff "in äquivalenter Weise produzierte" Antikörper wird verstanden, daß die Antikorper durch Immunisierung mit rekombinantem N-terminalem pro-BNP gewonnen werden.

Gegenstand der Erfindung sind auch Verfahren zur Herstellung von Antikörpern, die spezifisch N-terminales proBNP binden.

Die Herstellung von polyklonalen Antikörpern erfolgt bevorzugt mit den Schritten Immunisieren eines geeigneten Organismus wie beispielsweise Schafe mit rekombinant hergestelltem N-terminalem proBNP, Isolieren der Antikörper. Screenen nach den reaktivsten Epitopen und Aufreinigung der Antikörper über Immunsorption an geeigneten Peptiden. Ein solches Verfahren ist in Beispiel 2 beschrieben.

Die Herstellung von monoklonalen Antikörpern erfolgt bevorzugt mit den Schritten Immunisieren eines geeigneten Organismus wie beispielsweise Mäuse mit rekombinant hergestelltem N-terminalem proBNP und Auswahl der Klone bezüglich der Reaktivität der Antikörper mit nativem N-terminalem proBNP in verschiedenen Pools von Patientenseren. Ein solches Verfahren ist in Beispiel 3 beschrieben.

Die folgenden Beispiele erläutern die Erfindung weiter.

**Beispiel 1**

**Verfahren zur Herstellung von rekombinantem N-terminalen proBNP (1-76)**

1. Klonierung des rekombinanten N-terminalen proBNPs

Die Nukleotidsequenz des N-terminalen ProBNPs (Aminosäuresequenz 1-76) wurde mit Hilfe der Gensynthese hergestellt. Um eine in *E. coli* optimale Expression des Gens zu erreichen, wurde die DNA-Sequenz auf die in *E. coli* am häufigsten verwendeten Codons abgestimmt.Die Sequenzen der Oligonukleotide, die zur Herstellung des Gens verwendet wurden lauten wie folgt:

Pro5' (SEQ ID NO 1):

5'CCGGATCCCACCCGCTG3

Pro1hum (SEQ ID NO 2):

5'CGGGATCCCACCCGCTGGGTTCCCCGGGTTCCGCTTCCGACCTGGAAACCT CCGGTCTGCAGGAACAGCGTAACCACCT3'

Pro2hum (SEQ ID NO 3):

5'CGGTTCCAGGGAGGTCTGTTCAACCTGCAGTTCGGACAGTTTACCCTGCAG GTGGTTACGCTGTTCCTGC3'

Pro3hum (SEQ ID NO 4):

5'CAGACCTCCCTGGAACCGCTGCAGGAATCCCCGCGTCCGACCGGTGTTTGG AAATCCCGTGAAGTTGCTAC 3'

Pro4hum (SEQ ID NO 5):

5'CCCAAGCTTAACGCGGAGCACGCAGGGTGTACAGAACCATTTTACGGTGA
CCACGGATACCTTCGGTAGCAACTTCACGGGATTTCC3'

Pro3' (SEQ ID NO 6):

5' CCCAAGCTTAACGCGGAGC3'

Die Herstellung-des Gens erfolgte mit diesen Primern mit Hilfe der PCR (polymerase chain reaction). Das amplifizierte Gen wurde in einen geeigneten Vektor wie z.B. den Vektor pUC19 kloniert und sequenziert. Zur Klonierung des Gens in den Expressionsvektor pQE8 wurde das Gen über die Restriktionsschnittstellen *Bam* HI und *Hind* III aus dem Vektor pUC19 herausgeschnitten, in den Vektor pQE8 ligiert, der eine Expression von Proteinen mit N-terminalem Histidin-Tag erlaubt, und in *E. coli* M15 [pREP4] transformiert.

2. Expression des N-terminalen ProBNPs in *E. coli*

Zur Expression des Gens in *E. coli* wurde eine Übernachtkultur eines rekombinanten *E. coli* Klons 1/60 in Luria-Broth (mit 100 $\mu$g/ml Ampicillin und 50 $\mu$g/ml Kanamycin) überimpft und bei einer OD 550 von 1 mit IPTG (Isopropylthiogalactosid; 1 mM Endkonzentration) induziert. Nach der Induktion wurden die Kulturen noch weitere 4 Stunden bei 37°C inkubiert. Die Kulturen wurden abzentrifugiert und das Zellpellet in 50 mM Na-Phosphatpuffer, pH 8,0; 300 mM NaCl aufgenommen. Nach Aufschluss der Zellsuspension durch Ultraschall, wurde die Suspension abzentrifugiert und der Überstand auf eine Ni-NTA (Nitrilo-triacetat) Säule aufgetragen. Nach einem Waschschritt mit 50 mM Na-Phosphatpuffer, pH 8,0; 300 mM NaCl: 20 mM Imidazol wurde das Histidin-getaggte N-terminale proBNP eluiert mit 50 mM Na-Phosphatpuffer, pH 8,0; 300 mM NaCl; 300 mM Imidazol. Die eluierten Fraktionen wurden vereinigt und gegen 50 mM Tris pH 8,0 dialysiert. Zur Abtrennung von Verunreinigungen wurde das Dialysat auf eine Q-Sepharosesäule aufgetragen. Die Masse des aufgereinigten N-terminalen proBNPs wurde über MALDI-TOF bestimmt.

**Beispiel 2**

**Herstellung von polyklonalen Antikörpern gegen N-terminales pro-BNP**

1. Immunisierung

Schafe wurden mit rekombinantem N-terminalem proBNP(1-76) in kompletten Freund'schem Adjuvans immunisiert. Die Dosis betrug 0,1 mg je Tier. Die Immunisierungen wurden über 10 Monate im Abstand von 4 Wochen wiederholt. 6 Wochen nach der ersten Immunisierung und danach monatlich einmal wurden Serumproben gewonnen und auf Sensitivität und Titer untersucht.

2. Aufreinigung von polyklonalen Antikörpern aus Schafserum

Aus dem Rohserum eines mit rekombinantem N-terminalem proBNP immunisierten Schafs wurden Lipidbestandteile durch Delipidierung mit Aerosil (1,5%) entfernt. Danach wurden die Immunglobuline mit Ammoniumsulfat (2M) ausgefällt. Der gelöste Niederschlag wurde gegen 15mM $KPO_4$, 50mM NaCl pH 7.0 dialysiert und über DEAE-Sepharose chromatographiert. Die IgG-Fraktion, PAK<rec. NT-pro-BNP>S-IgG(DE), befand sich im Durchlauf.

3. Sequenzielle Affinitätschromatographie zur Herstellung von NT-pro-BNP spezifischen polyklonalen Antikörpern

Für die Aufreinigung von NT-proBNP spezifischen, gegen die Aminosäuren 1-21 gerichteten polyklonalen Antikörpern, PAK<rec. NT-pro-BNP>M-IgG(IS, 1-21), wurde das C-terminal biotinylierte Peptid HPLGSPGSASDLETSGLQEQR-Bi (1-21-Bi, SEQ ID NO 7) verwendet. Die Affinitätsmatrix wurde durch Beladen von 10ml Streptavidin beschichteten Methacrylatpolymer-Partikeln (Boehringer Mannheim, Best. Nr. 1529188) mit 1mg Peptid (1-21-Bi) hergestellt.
Mit 10ml der Affinitätsmatrix wurde eine Säule gepackt und mit 50mM $KPO_4$, 150mM NaCl pH 7,5 (PBS) äquilibriert. Für den ersten Schritt der sequenziellen Affinitätschromatographie wurden 850mg PAK<NT-pro-BNP>S-IgG(DE) auf die Säule aufgezogen. Der Durchlauf wurde für einen zweiten Schritt (siehe unten) aufgehoben. Die Säule wurde mit PBS und 20mM $KPO_4$, 500mM NaCl, 0,1% Triton X-100, 0,5% Na-Deoxicholsäure pH 7,5 gewaschen. Das spezifisch an die Affinitätsmatrix gebundene IgG wurde mit ImmunoPure® Gentle Ag/Ab Elution Buffer (Pierce. Product #: 21013) eluiert. Die Affinitätsmatrix wurde mit 1M Propionsäure regeneriert und in $PBS/NaN_3$ gelagert.

Auf die gleiche Art und Weise wie oben beschrieben, wurde das Peptid Bi-ELQVEQTSL (Bi-30-38 SEQ ID NO 8) für die Herstellung einer Affinitätsmatrix zur Aufreinigung von NT-pro-BNP spezifischen, gegen die Aminosäuren 30-38 gerichteten Immunoglobulinen verwendet. PAK<rec. NT-pro-BNP>M-IgG(IS,30-38) wurde aus dem Durchlauf der ersten Affinitätsreinigung gewonnen.

### 4. Biotinylierung von PAK<NT-pro-BNP>S-IgG(IS,1-21)

Die affinitätsgereinigten Antikörper werden gegen den Biotinviierungspuffer (100mM $KPO_4$, 70mM NaCl pH 8.0) dialysiert und anschließend wird die Lösung auf eine Proteinkonzentration von 1mg/ml eingestellt. D-Biotinoyl-Aminocapronsäure-N-Hydroxysuccinimidester wird in DMSO gelöst und in einem molaren Verhältnis von 1:7.5 der Antikörperlösung beigemischt. Die Reaktion wird durch Zugabe von L-Lysin gestoppt und das überschüssige Markierungsreagenz wird durch Dialyse entfernt.

### 5. Digoxigenylierung von PAK<NT-pro-BNP>S-IgG(IS,30-38)

Die affinitätsgereinigten Antikörper werden gegen den Digoxigenylierungspuffer (100mM $KPO_4$, 70mM NaCl pH 7,6) dialysiert und anschließend wird die Lösung auf eine Proteinkonzentration von 1 mg/ml eingestellt. Digoxigenin-3-CME-N-Hydroxy-succinimidester wird in DMSO gelöst und in einem molaren Verhältnis von 1:5 der Antikörperlösung beigemischt. Die Reaktion wird durch Zugabe von L-Lysin gestoppt und das überschüssige Markierungsreagenz wird durch Dialyse entfernt.

**Beispiel 3**

**Herstellung und Screening nach monoklonalen Antikörpern gegen N-terminales proBNP (1-76)**

### 1. Gewinnung von monoklonalen Antikörpern gegen NT-pro-BNP(1-76)

Balb/c-Mäuse, 8-12 Wochen alt, werden mit 100 $\mu$g rekombinantem N-terminalem proBNP-Antigen, mit komplettem Freundschen Adjuvans, intraperitoneal immunisiert. Nach 6 Wochen werden drei weitere Immunisierungen in 4 wöchigem Abstand durchgeführt. Eine Woche nach der letzten Immunisierung erfolgt eine Blutabnahme und die Bestimmung des Antikörpertiters im Serum der Versuchstiere. Aus positiv reagierenden Mäusen werden aus der Milz dieser Tiere die B-Lymphozyten gewonnen, die mit einer permanenten Myelomzellinie fusioniert werden. Die Fusionierung erfolgt nach dem bekannten Verfahren von Köhler und Millstein (Nature 256, 1975, S. 495 - 497). Die hierbei gebildeten Primärkulturen von Hybridzellen werden in üblicher Weise, z.B. unter Verwendung eines handelsüblichen Zellsorters oder durch "limiting dilution" kloniert. Es werden nur die Klonkulturen weiterverarbeitet, die in einem geeigneten Testverfahren, beispielsweise einem Enzym-Immuno-Assay (ELISA-Verfahren), positiv mit rekombinantem N-terminalem proBNP reagieren und natürliches N-terminales proBNP in Patientenseren erkennen (siehe Punkt 2.). Man erhält so mehrere Hybridoma-Zellinien, die die erfindungsgemäßen monoklonalen Antikörper produzieren.

Zur Erzeugung von Aszites werden 5 x $10^6$ Hybridomzellen intraperitoneal in Balb/c Mäuse gespritzt, die zuvor 1-2 mal mit 0,5 ml Pristan vorbehandelt worden sind. Nach 2-3 Wochen kann aus dem Bauchraum der Mäuse Aszites-Flüssigkeit gewonnen werden. Hieraus können in üblicher Weise die Antikörper isoliert werden. Diese monoklonalen Antikörper sind spezifisch gegen humanes N-terminales proBNP gerichtet. Sie werden im folgenden MAK M 10.1.11 bzw. MAK M 13.4.14 bezeichnet. Die beiden monoklonalen Antikörper gehören der Subklasse IgGl, kappa an.

Auf diese Weise konnten die beiden Hybridoma-Zellinien Klon M 10.1.11 und M 13.4.14 isoliert werden, die bei der DSMZ, wie oben erwähnt, hinterlegt wurden.

### 2. Screening-Test auf Antikörper gegen pro-BNP-Peptide und rekombinantes NT-pro-BNP

Um die Anwesenheit und Spezifität von Antikörpern gegen NT-proBNP im Serum immunisierter Mäuse, im Kulturüberstand der Hybridzellen oder in Aszitesflüssigkeit zu erkennen, wurden die Klone mit folgenden Testprinzipien bewertet:

a) Reaktivität mit rekombinantem N-terminalem proBNP

Mikrotiterplatten (Fa. Nunc, Maxisorb) werden mit 2,5 μg/ml rekombinantem NT-pro-BNP als Antigen in Beladungspuffer (Fa. Boehringer, 0,2 M Natriumcarbonat/-Bicarbonat, pH 9,3-9,5, Cat. No. 726 559) 100 μl/well, 1 Stunde bei Raumtemperatur unter Schütteln, gebunden. Die Nachbeladung erfolgt in PBS-Puffer (Phosphate Buffered Saline, Fa. Oxid, Code-BR 14a) und 1% Byco C. 30 Minuten. Anschließend wird mit Waschpuffer gewaschen (0,9 Natriumchlorid-Lösung, 0,05 % Tween 20). Die Antikörperprobeninkubation erfolgt mit 100 μl/well, 1 Stunde bei Raumtemperatur unter Schütteln. Dann wird erneut zweimal mit Waschlösung gewaschen. Dann erfolgt eine weitere Inkubation mit dem Nachweisantikörper PAK<M-Fcγ>S-Fab-Peroxidasekonjugates (Boehringer Mannheim, Kat. Nr. 1500 686), 100 mU/ml, 100 μl/well, 1 Stunde bei Raumtemperatur unter Schütteln. Nach einem erneuten Waschschritt mit Waschpuffer wird die Peroxidaseaktivität in üblicher Weise bestimmt (beispielsweise mit ABTS®, 30 Minuten bei Raumtemperatur, abgelesen wird die Extinktionsdifferenz in mE, bei 405 nm mittels eines ELISA-Readers.

b) Reaktivität mit N-terminalen proBNP-Peptiden:

In diesem Fall werden Streptavidin-beladene Mikrotiterplatten mit NT-pro-BNP-Peptid-Biotinkonjugaten der Sequenzen 1-10. 8-18, 1-21, 16-30, 30-38, 39-50. 50-63 oder 64-76 als Antigen, 250 ng/ml in PBS-Puffer (Phosphate Buffered Saline, Fa. Oxid, Code-BR 14a) mit 0,5 % Byco C , 100 μl/well, 1 Stunde bei Raumtemperatur unter Schütteln, gebunden. Anschließend wird mit Waschpuffer gewaschen (0.9 Natriumchlorid-Lösung, 0,05% Tween 20). Die Antikörperprobeninkubation und die Nachweisreaktion erfolgt wie unter a) beschrieben. Aufgrund der Reaktivität mit bestimmten NT-pro-BNP-Peptiden kann die Lage des Epitops erfaßt werden.

c) Reaktivität mit nativem N-terminalem proBNP in der Patientenprobe

Mikrotiterplatten (Fa. Nunc, Maxisorb) werden mit 5 μg/ml PAK<human pro-BNP>S-IgG (IS,(1-21) bzw. (30-38) S-IgG in Beschichtungspuffer(Fa. Boehringer, 0,2 M Natriumcarbonat/-Bicarbonat, pH 9,3 -9,5, Cat. No. 728 559) 100 μl/well, 1 Stunde bei Raumtemperatur unter Schütteln, gebunden. Die Nachbeladung erfolgt in PBS-Puffer (Phosphate Buffered Saline, Fa. Oxid, Code-BR 14a) und 1% Byco C, 30 Minuten. Anschließend wird mit Waschpuffer gewaschen (0,9 Natriumchlorid-Lösung, 0,05% Tween 20). Die Inkubation mit nativem Antigen in Patientenplasma, verdünnt in PBS-Puffer-erfolgt mit 100 μl/well. 1 Stunde bei Raumtemperatur unter Schütteln. Nach einem erneuten Waschschritt erfolgt die Antikörper-probeninkubation mit 100 μl/well, 1 Stunde bei Raumtemperatur unter Schütteln. Dann wird erneut zweimal mit Waschlösung gewaschen und es erfolgt eine weitere Inkubation mit dem Nachweisantikörper PAK<M-Fcγ>S-Fab-Peroxidase-konjugates (Boehringer Mannheim GmbH, Kat. Nr. 1500 686), 100 mU/ml, 100 μl/well, I Stunde bei Raumtemperatur unter Schütteln. Nach einem erneuten Waschschritt mit Waschpuffer wird die Peroxidaseaktivität in üblicher Weise bestimmt (beispielsweise mit ABTS®, 30 Minuten bei Raumtemperatur, abgelesen wird die Extinktionsdifferenz in mE, bei 405 nm mittels eines ELISA-Readers).

3. Ergebnisse: Reaktionsmuster der monoklonalen und polyklonalen Antikörper gegen N-terminales proBNP

a) Reaktivität der MAKs (c = 5 μg/ml) aus Immunisierung mit N-terminalen proBNP-Peptiden:

Tabelle 1:

| MAK | Immunogen | Reaktivität mit Pro-BNP-Peptiden | | | | | | | | Rec. pro-BNP | Natives pro-BNP |
|---|---|---|---|---|---|---|---|---|---|---|---|
| | | 1-10 | 8-18 | 1-21 | 16-30 | 30-38 | 39-50 | 50-63 | 64-76 | | |
| 5.2.27 | 1-10 | **1.42** | 0.04 | **1.48** | 0.05 | 0.03 | 0.04 | 0.04 | 0.04 | **1.16** | 0.30 |
| 2.1.4 | 16-30 | 0.04 | 0.04 | 0.04 | **1.86** | 0.04 | 0.04 | 0.04 | 0.04 | 0.1 | 0.02 |
| 1.2.6 | 39-50 | 0.04 | 0.04 | 0.03 | 0.04 | 0.03 | **1.23** | 0.03 | 0.04 | **0.44** | 0.06 |

Die monoklonalen Antikörper, die aus Immunisierungen mit unterschiedlichen Peptiden erhalten wurden, reagieren sehr stark mit den jeweiligen Peptiden. Die Reaktivität mit dem rekombinanten N-terminalen proBNP ist nur bei 2 monoklonalen Antikörpern zu erkennen, während mit nativem N-terminalen proBNP in einem Patientenpool keine Reaktion erfolgt (siehe Tabelle 1).

b) Reaktivität der monoklonalen Antikörper (MAK) aus Immunisierung mit rekombinantem N-terminalem proBNP:

Tabelle 2:

| MAK | Reaktivität mit Pro-BNP-Peptiden | | | | | | | | Rec. pro-BNP | Natives proBNP |
|---|---|---|---|---|---|---|---|---|---|---|
| | 1-10 | 8-18 | 1-21 | 16-30 | 30-38 | 39-50- | 50-63 | 64-76 | | |
| 10.1.11 | 0.04 | **0.97** | **1.03** | 0.04 | 0.04 | 0.06 | 0.04 | 0.04 | **1.61** | **1.70** |
| 10.3.19 | 0.04 | 0.04 | 0.04 | 0.04 | 0.04 | 0.05 | 0.04 | 0.03 | **1.24** | **0.91** |
| 10.3.30 | 0.04 | 0.04 | 0.03 | 0.04 | 0.04 | 0.06 | 0.04 | 0.03 | **1.43** | **0.79** |
| 13.4.14. | 0.04 | 0.04 | 0.04 | 0.04 | 0.04 | 0.05 | 0.03 | 0.04 | **1.65** | **1.83** |
| 13.1.18 | 0.04 | 0.04 | 0.03 | 0.04 | **1.14** | 0.03 | 0.04 | 0.04 | **1.47** | **0.56** |
| 13.2.22 | 0.04 | 0.04 | 0.04 | 0.0:1 | 0.04 | 0.03 | 0.04 | 0.04 | **1.82** | **1.61** |

Die monoklonalen Antikörper aus der Immunisierung mit rekombinantem N-terminalen proBNP reagieren nur vereinzelt mit Peptiden, aber sehr stark mit rekombinantem N-terminalen proBNP bzw. mit nativem N-terminalen proBNP in einem Patientenpool. Die Nichtreaktion einzelner monoklonaler Antikörper mit den Peptiden deutet auf die Erkennung sogenannter Konformationsepitope hin (siehe Tabelle 2).

c) Reaktivität der PAKs aus Immunisierung mit rekombinantem N-terminalem proBNP:

Tabelle 3:

| PAK | Immun-sorption | Reaktivität mit Pro-BNP-Peptiden | | | | | | | | Rec. proBNP | Natives pro-BNP |
|---|---|---|---|---|---|---|---|---|---|---|---|
| | | 1-10 | 18-18 | 1-21 | 16-30 | 30-38 | 39-50 | 50-63 | 64-76 | | |
| S-9212 | ohne | 0.13 | **1.81** | **1.98** | **1.16** | **2.99** | **0.83** | **1.22** | 0.06 | **0.89** | - |
| S-9212 | 1-21 | **0.99** | **2.99** | **2.99** | **1.00** | 0.20 | 0.13 | 0.20 | 0.15 | **1.98** | **1.41** |
| S-9212 | 30-38 | 0.08 | 0.07 | 0.07 | 0.07 | **2.99** | 0.06 | 0.17 | 0.06 | **2.99** | **1.41** |

Der gewonnene PAK reagierte am stärksten mit den Peptiden 1-21 und 30-38. Aus diesem Grund wurden diese Epitope ausgewählt und der PAK mit Hilfe dieser Peptide positiv immunsorbiert. Der mit Peptid 1-21 immunsorbierte PAK reagiert am stärksten mit der Region 8-20 und deutlich abgeschwächt mit der N-terminalen Sequenz 1-10. Die so immunsorbierten PAKs reagieren sehr stark mit dem rekombinanten N-terminalen proBNP und im PAK/PAK-Sandwichformat mit der nativen Probe (siehe Tabelle 3).

**Beispiel 4**

**Hochsensitiver Immunoassay zur Bestimmung von NT-pro-BNP**

Mit Hilfe der in Beispiel 2 und 3 gewonnenen Antikörper konnte ein hochsensitiver Immunoassay aufgebaut werden. Generell sind alle Testformate geeignet, bei denen 2 Antikörper mit unterschiedlicher Epitoperkennung eingesetzt werden. Als Beispiel wird ein sogenannter Sandwich-ELISA beschrieben.

Als Festphase wurde eine mit Streptavidin beschichtete Mikrotiterplatte (MTP) verwendet. 10 $\mu$l unbehandelte Probe oder Kalibrator wird zusammen mit 100 $\mu$l Puffer, der die beiden epitop-spezifischen Antikörper enthält, in die MTP-Näpfe pipettiert und 1 Stunde bei Raumtemperatur inkubiert. Als Antikörper wurden 1 $\mu$g/ml biotinylierter PAK<rec.

NT-pro-BNP>S-IgG(IS,1-21) und 0.5 µg/ml digoxigenylierter PAK< rec. NT-pro-BNP>S-IgG(IS,30-38) eingesetzt. Anschließend wird die Lösung abgesaugt und 3x mit 350 µl Waschpuffer gewaschen. Danach werden 100 µl Konjugatösung dazupipettiert und wiederum 1 h bei Raumtemperatur inkubiert. Als Konjugat wird ein Anti-Digoxin-Antiköper-POD-Konjugat in einer Konzentration von 100 mIU/ml verwendet. Anschließend wird die Konjugatlösung abgesaugt und 3x mit 350 µl Waschpuffer gewaschen. Zum Schluß wird ABTS®-Substratlösung in die Wells pipettiert und 30 Minuten bei Raumtemperatur gemessen. Nach Erreichen der 30-minütigen Substratreaktion wird die Mikrotiterplatte sofort in einem MTP-Reader bei einer Wellenlänge von 405 nm gegen die Referenzwellenlänge von 495 nm vermessen.

Zur Bestimmung der Sensitivität wurde eine Eichkurve erstellt und die Präzision des Nullstandards (n = 21) bestimmt. Als Kalibratoren wurde humanes EDTA-Plasma verwendet, welches mit rekombinantem N-terminalen proBNP in der erforderlichen Konzentration aufgestockt wurde. Als Nullstandard wurde Rinderplasma verwendet. Die Ergebnisse sind in Tabelle 4 dargestellt.

Tabelle 4:

|  | Extinktion (Mittelwert) | Standardabweichung (n=21) |
|---|---|---|
| Kalibrator a: 0 fmol/ml | 131 mE | 5.7 mE |
| Kalibrator b: 5.04 fmol/ml | 268 mE | |
| Kalibrator c: 19.9 fmol/ml | 746 mE | |
| Kalibrator d: 50.5 fmol/ml | 1500 mE | |
| Kalibrator e: 100.9 fmol/ml | 2401 mE | |

Anhand der Eichkurvensteilheit von 22.5 mE x ml/fmol und einem SD von 5,7 mE ergibt sich nach der Formel von Kaiser folgende untere Nachweisgrenze:

$$ UNG = 3 \ SD_{Nullstandard}/Ek\text{-}steilheit = 3 \times 5.7/22.5 = 0.76 \ fmol/ml. $$

## Beispiel 5

### Ermittlung der Probenstabilität von N-terminalem proBNP

Mit Hilfe des in Beispiel 4 beschriebenen Sandwich-ELISA wurde die Analytstabilität von N-terminalem proBNP vermessen. Dazu wurden 4 Patienten mit NYHA-Klasse II-III Blut in EDTA-haltigen Abnahmeröhrchen abgenommen und bei Raumtemperatur über 3 Tage aufbewahrt. Jeden Tag wurde eine Probe entnommen und der Gehalt an N-terminalem proBNP vermessen. Die Referenzprobe sowie die Proben zur Stabilitätsermittlung in EDTA-Plasma wurden sofort auf 4°C - 8°C abgekühlt und innerhalb von 15 Minuten zentrifugiert. Die EDTA-Plasmen wurden bei 4°C und Raumtemperatur aufbewahrt und zu verschiedenen Zeitpunkten innerhalb einer 24-stündigen Belastungszeit vermessen. Die Ergebnisse sind in der Tabelle 5 dargestellt.

Tabelle 5:

| Belastungzeit | | Wiederfindung (%) |
|---|---|---|
| EDTA-Vollblut, Raumtemperatur | 24 h | 98.8 |
| | 48 h | 98.0 |
| | 72 h | 100.5 |
| EDTA-Plasma, 4°C | 2 h | 97.5 |
| | 4 h | 98.5 |
| | 6 h | 102.0 |
| | 24 h | 103.0 |
| EDTA-Plasma. Raumtemperatur | 2 h | 103.0 |
| | 4 h | 104.8 |

(fortgesetzt)

| Belastungzeit | Wiederfindung (%) |
|---|---|
| 6 h | 102.0 |
| 24 h | 96.0 |

Diese Daten belegen, daß N-terminales proBNP innerhalb der geprüften Zeitpunkte vollkommen stabil ist und somit als Routineparameter verwendbar ist. Dieses Ergebnis ist widersprüchlich zur Literatur (Hunt et. al., Clinical Endocrinology, 47, 287 (1997)) und bestätigt die Annahme, daß durch Auswahl und Design dieses Testformats mit 2 spezifischen Antikörpern, deren Epitope nicht am äußeren Ende des Analyten liegen, die Analytstabilität beeinflußt werden kann.

**Beispiel 6**

**Ermittlung der diagnostischen Sensitivität des N-terminalen proBNP-Assays**

Zur Ermittlung der diagnostischen Sensitivität wurde wiederum der in Beispiel 4 beschriebene Test verwendet. Dazu wurden 1 14 Gesunde und 235 Patienten mit einer NYHA-Klassifizierung zwischen I und IV vermessen. Normalerweise ist es besonders kritisch, Gesunde von Patienten mit NYHA-Klasse I zu unterscheiden.

Mit diesem hochsensitiven Assay wurde bei den 110 gesunden Blutspendern ein Medianwert von 6,6 fmol/ml NT-proBNP mit einer Standardabweichung von 7,3 fmol/ml ermittelt. Der niedrigste Wert wurde mit 0,2 fmol/ml ermittelt. Dies zeigt deutlich, daß eine Sensitivität < 1,0 fmol/ml notwendig ist, um den Referenzbereich genau zu erfassen. Mit Hilfe dieser Verteilung wurde der obere Normalwertbereich (97.5% Percentile) mit 26.6 fmol/ml ermittelt.

Unter Annahme des Referenzwertbereichs von 0 - 26.6 fmol/ml wurden von den 233 Patienten mit NYHA-Klassifizierung I-IV nur 16 Patienten mit einem Wert im Normalbereich ermittelt. Dies entspricht einer klinischen Sensitivität von 93.3%. Werden nur die Patienten mit einer NYHA-Klassifizierung von I betrachtet, dann werden 30 der 37 Patienten positiv erkannt, dies entspricht einer Sensitivität von 81,1%.

Dieses Ergebnis bestätigt, daß durch diesen hochsensitiven N-terminalen proBNP-Assay eine deutliche Differenzierung zwischen Patienten mit einer Herzinsuffizienz der NYHA-Klasse 1 von gesundem Normalkollektiv unterscheidbar ist. Dies konnte mit den bisher im Stand der Technik verfügbaren Assays (Dagubatti et al., Cardiovascular Research 36 (1997), 246 nicht erreicht werden.

**Patentansprüche**

1. Verfahren zum Nachweis von nativem N-terminalen proBNP in einer Probe, das im Sandwichformat durchgeführt wird, mit mindestens zwei Antikörpern, die verschiedene Epitope des nativen N-terminalen proBNP erkennen und gleichzeitig am nativen N-terminalen proBNP binden können, **dadurch gekennzeichnet, dass** die mindestens zwei Antikörper im Bereich von Aminosäure 10-50 des N-terminalen proBNP binden und die Antikörper durch Immunisieren eines geeigneten Organismus mit rekombinantem NTproBNP erhalten werden.

2. Verfahren nach Anspruch 1, **dadurch gekennzeichnet, dass** die Nachweisgrenze für N-terminales proBNP unter 1 fmol/ml eingesetzter Probe liegt.

3. Verfahren nach Anspruch 1 oder 2 **dadurch gekennzeichnet, dass** mittels der erhaltenen Werte eine Differenzierung nach Proben von gesunden Patienten und Proben von Patienten mit Herzinsuffizienz der NYHA-Klassen I bis IV vorgenommen werden kann.

4. Verfahren nach einem der vorhergehenden Anspruche **dadurch gekennzeichnet, dass** anhand der erhaltenen Werte eine Differenzierung zwischen Proben von gesunden Patienten und Proben von Patienten der NYHA-Klasse I vorgenommen werden kann.

5. Verwendung des Verfahrens gemäß einem der vorhergehenden Ansprüche zur Differenzierung zwischen Proben von gesunden Patienten und Proben von Patienten der NYHA-Klassen I bis IV.

6. Verwendung von rekombinantem N-terminalen proBNP als Standard in einem Verfahren zum Nachweis von N-terminalem proBNP gemäß den Ansprüchen 1 bis 4.

7. Antikörper gegen N-terminales proBNP, hergestellt durch Immunisieren eines geeigneten Organismus mit rekombinant hergestelltem N-terminalem proBNP, **dadurch gekennzeichnet, dass** sie im Bereich von Aminosäure 10 bis 50 des N-terminalen proBNP spezifisch binden.

8. Antikörper gemäß Anspruch 7, erhältlich aus den am 26.01.1999 bei der DSMZ hinterlegten Zellinien M 10.1.11 (DSM ACC 2386) oder M 13.4.14 (DSM ACC 2387).

9. Antikörper gemäß Anspruch 8, die in äquivalenter Weise mit N-terminalem proBNP hergestellt wurden wie die Antikörper, die von den Zellinien M 10.1.11 (DSM ACC 2386) oder M 13.4.14 (DSM ACC 2387), produziert werden.

10. Zellinien M 10.1.11 (DSM ACC 2386) oder M 13.4.14 (DSM ACC 2387) hinterlegt am 26.01.1999 bei der DSMZ.

11. Verfahren zur Herstellung von polyklonalen Antikörpern gemäß den Ansprüchen 7 oder 9, enthaltend die Schritte Immunisieren eines geeigneten Organismus mit rekombinant hergestelltem N-terminalem proBNP, Isolieren der Antikörper, Screenen nach den reaktivsten Epitopen und Aufreinigung der Antikörper über Immunsorption an geeigneten Peptiden.

12. Verfahren zur Herstellung von monoklonalen Antikörpern gemäß den Ansprüchen 7 bis 9, enthaltend die Schritte Immunisieren eines geeigneten Organismus mit rekombinant hergestelltem N-terminalem proBNP und Auswahl der Klone bezüglich der Reaktivität der Antikörper mit nativem N-terminalem proBNP in verschiedenen Pools von Patientenseren.

**Claims**

1. Method for detecting native N-terminal proBNP in a sample which is carried out in a sandwich format using at least two antibodies which recognize different epitopes of the native N-terminal proBNP and at the same time can bind to native N-terminal proBNP, **characterized in that** the at least two antibodies bind in the region of amino acid 10-50 of N-terminal proBNP and the antibodies are obtained by immunizing a suitable organism with recombinant NTproBNP.

2. Method according to claim 1, **characterized in that** the detection limit for N-terminal proBNP is below 1 fmol/ml of sample used.

3. Method according to claim 1 or 2, **characterized in that** the values obtained can be used to differentiate between samples from healthy patients and samples from patients with heart failure of NYHA classes I to IV.

4. Method according to one of the previous claims, **characterized in that** a differentiation between samples of healthy patients and samples of patients of the NYHA class I can be carried out on the basis of the values obtained.

5. Use of the method according to one of the previous claims to differentiate between samples of healthy patients and samples of patients of NYHA classes I to IV.

6. Use of recombinant N-terminal proBNP as a standard in a method for detecting N-terminal proBNP according to claims 1 to 4.

7. Antibodies against N-terminal proBNP produced by immunizing a suitable organism with recombinantly produced N-terminal proBNP, **characterized in that** they specifically bind in the region of amino acid 10 to 50 of N-terminal proBNP.

8. Antibodies according to claim 7 obtainable from the cell lines M 10.1.11 (DSM ACC 2386) or M 13.4.14 (DSM ACC 2387) deposited on 26.01.1999 at the DSMZ.

9. Antibodies according to claim 8 which were produced in an equivalent manner using N-terminal proBNP to the antibodies which are produced by the cell lines M 10.1.11 (DSM ACC 2386) or M 13.4.14 (DSM ACC 2387).

10. Cell lines M 10.1.11 (DSM ACC 2386) or M 13.4.14 (DSM ACC 2387) deposited on 26.01.1999 at the DSMZ.

11. Process for producing polyclonal antibodies according to claims 7 or 9 comprising the steps immunizing a suitable organism with recombinantly produced N-terminal proBNP, isolating the antibodies, screening for the most reactive epitopes and purifying the antibodies by immunoadsorption to suitable peptides.

12. Process for producing monoclonal antibodies according to claims 7 to 9 comprising the steps immunizing a suitable organism with recombinantly produced N-terminal proBNP and selecting the clones with regard to the reactivity of the antibodies with native N-terminal proBNP in different pools of patient sera.

**Revendications**

1. Procédé de détection de proBNP N-terminal natif dans un échantillon qui est réalisé dans un format en sandwich, avec au moins deux anticorps qui détectent différents épitopes du proBNP N-terminal natif et qui peuvent simultanément se lier au proBNP N-terminal natif, **caractérisé en ce que** lesdits au moins deux anticorps se lient dans la zone des aminoacides 10-50 du proBNP N-terminal et **en ce que** les anticorps sont obtenus par immunisation d'un organisme approprié avec du NTproBNP recombinant.

2. Procédé selon la revendication 1, **caractérisé en ce que** la limite de détection pour le proBNP N-terminal se situe en dessous de 1 fmole/ml d'échantillon utilisé.

3. Procédé selon la revendication 1 ou 2, **caractérisé en ce qu'**au moyen des valeurs obtenues, on peut réaliser une différenciation des échantillons de patients sains et d'échantillons de patients souffrant d'une insuffisance cardiaque des classes NYHA I à IV.

4. Procédé selon l'une quelconque des revendications précédentes, **caractérisé en ce qu'**à l'aide des valeurs obtenues, on peut réaliser une différenciation entre des échantillons de patients sains et des échantillons de patients de la classe NYHA 1.

5. Utilisation du procédé selon l'une quelconque des revendications précédentes pour la différenciation entre des échantillons de patients sains et des échantillons de patients des classes NYHA I à IV.

6. Utilisation de proBNP N-terminal recombinant comme standard dans un procédé pour la détection de proBNP N-terminal selon les revendications 1 à 4.

**7.** Anticorps contre le proBNP N-terminal préparés par immunisation d'un organisme approprié avec du proBNP N-terminal préparé de manière recombinante, **caractérisés en ce qu'**ils se lient spécifiquement dans la zone des aminoacides 10 à 50 du proBNP N-terminal.

**8.** Anticorps selon la revendication 7, pouvant être obtenus à partir des lignées cellulaires M 10.1.11 (DSM ACC 2386) ou M 13.4.14 (DSM ACC 2387) déposées le 26.01.1999 auprès de la DSMZ.

**9.** Anticorps selon la revendication 8, qui ont été préparés de manière équivalente avec du proBNP N-terminal aux anticorps qui ont été produits par les lignées cellulaires M 10.1.11 (DSM ACC 2386) ou M 13.4.14 (DSM ACC 2387).

**10.** Lignées cellulaires M.10.1.11 (DSM ACC 2386) ou M 13.4.14 (DSM ACC 2387) déposées le 26.01.1999 auprès de la DSMZ.

**11.** Procédé pour la préparation d'anticorps polyclonaux selon les revendications 7 ou 9, contenant les étapes d'immunisation d'un organisme approprié avec du proBNP N-terminal préparé de manière recombinante, d'isolement des anticorps, de criblage des épitopes les plus réactifs et de purification des anticorps par immunosorption sur des peptides appropriés.

**12.** Procédé pour la préparation d'anticorps monoclonaux selon les revendications 7 à 9, contenant les étapes d'immunisation d'un organisme approprié avec du proBNP N-terminal préparé de manière recombinante et de choix des clones en ce qui concerne la réactivité des anticorps avec du proBNP N-terminal natif dans différents pools de sérums de patients.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0542255 A
- WO 9324531 A
- US 5786163 A
- EP 0186799 A

**In der Beschreibung aufgeführte Nicht-Patentliteratur**

- *Clin. Sci,* 1998, vol. 95 (3), 235-239
- **CLELAND et al.** *Heart,* 1996, vol. 75, 410-413
- **SUDOH et al.** *Nature,* 1988, vol. 332, 78-81
- **MASUTA et al.** *Clin. Chem.,* 1998, vol. 44 (6), 130
- **TSUJI et al.** *Clin. Chem.,* 1994, vol. 40, 672
- **HUNT et al.** *Biochem. Biophys. Res. Com,* 1995, vol. 214, 1175-1183
- **HUNT et al.** *Peptides,* 1997, vol. 18 (10), 1475-1481
- **HUNT et al.** *Clinical Endocrinology,* 1997, vol. 47, 287-296
- **KÖHLER ; MILLSTEIN.** *Nature,* 1975, vol. 256, 495-497
- **HUNT.** *Clinical Endocrinology,* 1997, vol. 47, 287
- **DAGUBATTI et al.** *Cardiovascular Research,* 1997, vol. 36, 246

SEQUENZ PROTOKOLL

<110> Roche Diagnostics GmbH

<120> Verfahren zum Nachweis von N-terminalem proBNP

<130> 51810AWO-SZ

<140>
<141>

<160> 6

<170> PatentIn Ver. 2.1

<210> 1
<211> 17
<212> DNA
<213> E. coli

<400> 1
ccggatccca cccgctg          17

<210> 2
<211> 79
<212> DNA
<213> E. coli

<400> 2

```
cgggatccca cccgctgggt tccccgggtt ccgcttccga cctggaaacc tccggtctgc 60
aggaacagcg taaccacct                                                79
```

<210> 3
<211> 70
<212> DNA
<213> E. coli

<400> 3

```
cggttccagg gaggtctgtt caacctgcag ttcggacagt ttaccctgca ggtggttacg 60
ctgttcctgc                                                          70
```

<210> 4
<211> 71
<212> DNA
<213> E. coli

<400> 4

```
cagacctccc tggaaccgct gcaggaatcc ccgcgtccga ccggtgtttg gaaatcccgt 60
gaagttgcta c                                                        71
```

<210> 5
<211> 87
<212> DNA
<213> E. coli

<100> 5

```
cccaagctta acgcggagca cgcaggtgt acagaaccat tttacggtga ccacggatac 60
cttcggtagc aacttcacgg gatttcc                                       87
```

<210> 6
<211> 19
<212> DNA
<213> E. coli

<400> 6
cccaagctta acgcggagc        19